# EUROPEAN PATENT SPECIFICATION

(11) **EP 1 824 561 B1**
(45) Date of publication and mention of the grant of the patent: **13.04.2016**
(21) Application number: 05769720.3
(22) Date of filing: 05.08.2005
(51) Int. Cl.: A61N 5/06

(54) **PHOTOCOSMETIC METHOD**
PHOTOKOSMETISCHES VERFAHREN
PROCEDE PHOTOCOSMETIQUE

(30) Priority: 05.08.2004 GB 0417506; 27.04.2005 GB 0508558
(43) Date of publication of application: 29.08.2007
(73) Proprietor: Photo Therapeutics Limited, Altrincham Cheshire WA14 1EP (GB)
(72) Inventor: WHITEHURST, Colin, Altrincham, Cheshire WA14 1EP (GB)
(74) Representative: Cross, James Peter Archibald
(86) International application number: PCT/GB2005/003101
(87) International publication number: WO 2006/013390

(56) References cited:
- WO-A-95/19809
- WO-A-99/19024
- CA-C- 2 112 132

## Description

### Field of the Invention

The present invention relates to method of phototherapy for skin rejuvenation, enhancing aesthetic treatments.

### Background of the Invention

The application of non-ablative skin rejuvenation to repair, or offset, the results of both chronological- and photo-ageing in the skin of the face, neck, hands and exposed areas of the body has become extremely popular. It is now one of the fastest growing procedures for dermatological and aesthetic clinics. From the original mechanical and chemical peels, clinicians progressed very swiftly to the use of ablative skin rejuvenation using lasers, but with the negative effects of severe morbidity (erythema and oedema) resulting in patient downtime. These disadvantages significantly offset the good results of the treatment. Lasers and Intense Pulsed Light (IPL) sources were then developed to deliver thermal damage to the dermis under cooling, termed non-ablative skin rejuvenation. This generally localised thermal damage to the deeper layers of the skin (dermis) whilst the forced cooling helped to protect the upper (and therefore highly visible) layers from thermal damage.

However, instead of treating the symptoms of chronological- and photo-ageing it would be desirable to prevent or halt them at an early stage, or even treat their causes through the use of phototherapeutic techniques which are less invasive or damaging than ablative or even non-ablative skin rejuvenation using laser and IPL sources.

Good and predictable wound healing is an essential part of plastic surgery. Occasionally, however, a wound does not heal as well as it should and this can cause problems such as hypertrophic or atrophic scarring or chronic ulceration. For the plastic surgeon, and for the patient, these are unacceptable outcomes.

Laser ablative skin resurfacing has been a popular modality for the removal or improvement of major wrinkles and other severe symptoms of aging. The principles of ablative therapy are based on light-tissue interaction delivering the optimum amount of controlled residual thermal damage with precise epidermal ablation, therefore invoking a wound response and thus maximising the clinical result whilst minimising side effects and their associated downtime. Unfortunately, the resulting crusting, oedema and long-term erythema are major stumbling blocks for all but the most determined patient.

Hence, there is a need to accelerate and/or improve healing of the skin following surgery or other invasive or non-invasive treatment. Accelerated surgical recovery offers enhanced patient safety (e.g. a reduced window for infection and pain) as well as fitting with the trend towards less aggressive and less invasive treatments.

It has been proposed to accelerate and/or improve wound healing by phototherapy, in which light at a specific wavelength is absorbed by molecules known as photoacceptors. These photoacceptors can either be exogenous, as in the case of aminolaevulinic acid based Photodynamic therapy (ALA-PDT), or endogenous where they occur naturally in the body. Light is absorbed by a photoacceptor and modulates the behaviour of the photo acceptor or cellular substrate causing a cascade of biochemical events. This then evokes a range of a cellular responses including cellular proliferation or modulation of the particular function of the cell, or the repair of damaged or compromised cells. This was formerly termed "biostimulation", but because some of the reactions can result in the retardation of bioprocesses in addition to acceleration, more correct terms would be "photobiomodulation" or "photoimmune modulation". This group of reactions is generated by the chemical and physical changes which occur as a result of the action of light absorption by photoacceptors. Light absorption and the resultant reactions are highly wavelength-specific, so the selection of wavelength is important when attempting to achieve specific reactions.

The evolution of the therapeutic light-emitting diode (LED) with narrow bandwidths has offered the aesthetic dermatologist a new tool that can target specific cellular chromophores or acceptors in the skin tissue and thereby initiate a cascade of natural biological processes/metabolism which revitalise/improve/regenerate/stimulate the functionality and appearance of the skin. LEDs can be arranged in arrays developed and designed to deliver precise doses of phototherapeutic energy over comparatively short periods of time.

US patent no. 5,800,479 and publication WO 95/19809 describe methods of treatment of wounds or sores using pulsed infrared and visible light emitted by an LED array. In one example, the pulsed infrared and visible light alternate over a period of between one and three minutes. The preferred wavelength of the visible light is 660 nm.

### Statement of the Invention

Aspects of the present invention are set out in the accompanying claims.

### Brief Description of the Drawings

Specific embodiments of the present invention will now be described with reference to the accompanying drawings, in which:
Figure 1 is a schematic diagram comparing the action mechanisms of visible and near 1R light on target cells;
Figure 2 is a diagram illustrating the population of different types of cell during inflammation, proliferation and remodelling phases;
Figure 3 is a table showing the action of different wavelengths on the different types of cell shown in Figure 2;
Figure 4 is a flow diagram of a course of phototherapy;
Figure 5 is a flow diagram of courses of phototherapy before and after a treatment;
Figure 6 is a flow diagram of courses of phototherapy in an embodiment of the present invention;
Figure 7 is schematic side view of a light source suitable for use in embodiments of the invention;
Figure 8a is a front perspective view of a light-emitting head of the light source showing panels each carrying an LED matrix;
Figure 8b is a top view of the light-emitting head showing the direction of illumination;
Figure 9 is a front view of one of the LED matrices; and
Figure 10 is a circuit diagram showing the series-parallel configuration of each LED matrix.

### Detailed Description of Embodiments

### Theory

To facilitate understanding of embodiments of the invention, the underlying theory behind "photobiomodulation" or "photoimmune modulation" will first be described.

### Primary and Secondary Reactions

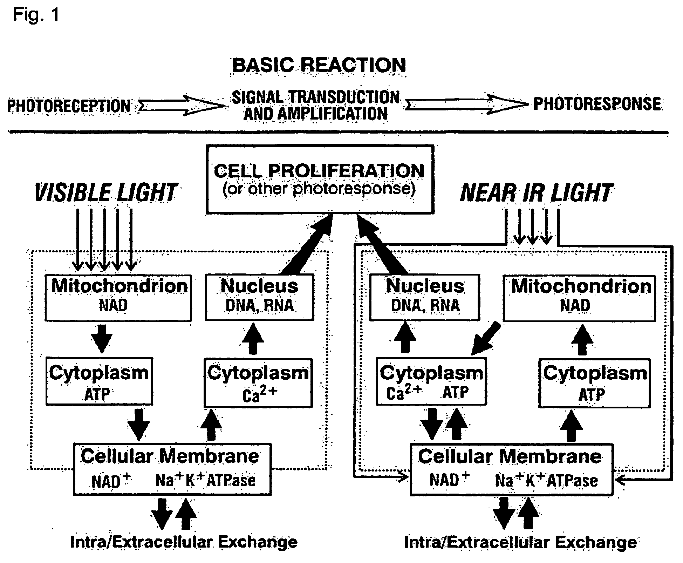

When a cellular organelle, substrate or cellular membrane receptor absorbs a photon from the visible region of the spectrum, there are two distinct types of cellular reaction. There is a primary photochemically-mediated reaction that occurs immediately and is usually comprised of changes in the redox chain within the cell, *i.e.* the cellular respiratory process that stimulates a number of other cellular processes as shown in Figure 1, and there is a secondary reaction that occurs after light therapy. These reactions involve the cellular pathways that regulate cell homeostasis and cell proliferation.

### Wavelengths

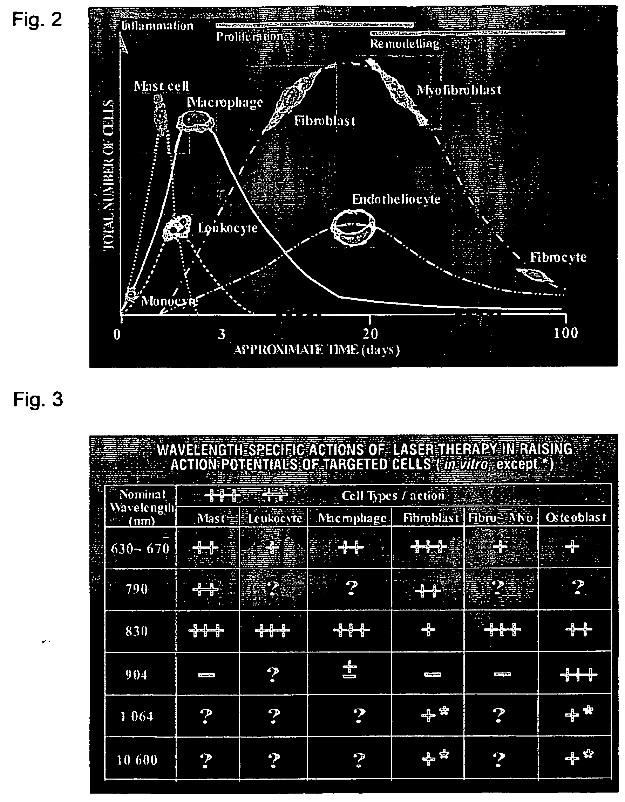

In phototherapy, visible and near-infrared light are the main wavelengths used; it is necessary to know what specific targets to aim at within the tissue. The effectiveness of different wavelengths in targeting different types of cell is illustrated in Figure 2. If targeting cells called fibroblasts, which synthesise the collagen, then red light is most effective. This is most useful in wound healing and in encouraging collagenesis in the photorejuvenation of photoaged skin. If targeting the inflammatory process cells, such as leukocytes and mast cells in the first inflammatory stages of wound healing, and macrophage cells in the second proliferative and early third modelling stages, then near-infrared light of around 830nm is most effective. Also, in pain therapy, when it is necessary to get photons deep into the major joints such as the shoulder, elbow, hip, knee and ankle joints, then the better penetration of infrared light allows a deeper delivery of photons to the target cell.

Each wavelength has a specific target in the living cell, and these targets are known as chromophores, or photoacceptors. A typical cell consists of a nucleus in the middle of the cell, containing the DNA from which the mother cell can construct similar daughter cells in the process known as replication. Around the periphery of the cell is the cellular membrane, which encloses the cell and protects it from the external environment. The fluid within the cell called the cytoplasm, and in this float a number of important subcellular organelles, such as ribosomes (RNA factories), lysosomes (enzyme factories) and most important, mitochondria (power houses of the cells, and manufacturers and regulators of adenine triphosphate (ATP), the fuel of the cell.

When a molecule absorbs a photon of light from the visible region, the electrons of that molecule are raised to a higher energy state. This excited molecule must then lose its extra energy. This loss of energy is achieved by:
a) Re-emitting a photon of longer wavelength (less energy) for example in fluorescence or phosphorescence,
   or
b) Undergoing photochemistry (absorption of non-ionising electromagnetic radiation).

There are four primary mechanisms that can occur simultaneously and which explain the action of light on photoacceptors and the subsequent cellular processes that are seen.
1. Acceleration of electron transfer in the intracellular respiratory chain
2. Changes in biochemical pathways, induced by transient heating of chromophores
3. Production of superoxide radicals
4. Singlet oxygen production or localised photodynamic therapy

For visible red light (620 nm - 680 nm), the main photoacceptors are found inside the mitochondria, on that part of the mitochondrial respiratory system which controls the energy level or metabolism of the cell called the redox chain. When visible light photons are absorbed by the redox chain, they transfer their energy to the respiratory system: when enough photons are absorbed, a photomodulated cascade of events is triggered within the cell, and the energy level of the entire cell is dramatically increased. This in turn changes the outer membrane permeability, and the now-excited cell exchanges energy-charged particles from its cytoplasm, especially calcium ions (Ca²⁺) and protons (H⁺) with other nearby cells via the extracellular environment. Thus, even unirradiated cells are excited by the messages they get from irradiated cells. The target cells are thus photoactivated, and either cell replication occurs faster, or the cells are stimulated into doing their job better. For example, photomodulated fibroblasts will produce more and better collagen fibres. Visible red light can thus be said to work from the inside of cells to the outside.

For invisible, near-infrared light (750 nm - 1070 nm), on the other hand, the main photoacceptors are found firstly in the outer membrane of the cell, and secondly on the membranes of the subcellular organelles. When enough energy has been absorbed, the cell membrane permeability changes, more calcium ions and protons are produced in the cytoplasm, and more protons.can penetrate into the cell, raising the energy level of the mitochondria through acting directly on their membranes. The same photocascade of events is thus set in motion as in the case of the red visible light photons, but from a different stage, so that there is actually a double action with infrared light. Thus the same end point is reached with IR photomodulation, namely cell proliferation, or the action potential of the cell is modulated. IR light can thus be said to work from the outside of the cell to the inside, and then back out again. The fact that cells have specific photoacceptors thus explains why red light is best for modulating fibroblast function, whereas IR light is best for leukocytes and macrophages in inflammation control, and neurons (nerve cells) in pain control. However, IR light will still have some photocybomodulatory effect on fibroblasts, and red light will still have some photocybomodulatory effect on inflammatory and nerve cells, particularly those located more superficially, as IR photons can penetrate much more deeply than visible red photons.

### Intracellular light reactions to red light at 633 nm

TI Karu, "Photobiology of low power laser effects. " Health Phys. 1989; 56:691-704 proposed a cascade of molecular events initiated by the absorption of photons of light by primary photoacceptors, NADH dehydrogenase and cytochrome c oxidase, contained within the mitochondria causing a transient activation of the respiratory chain and the formation of transient superoxide radicals. Importantly one of the key functions of the respiratory chain is to control cellular homeostasis. The resultant proton gradient between the mitochondrial matrix and cytosol results in an electrochemical potential across the inner membrane of the mitochondrial matrix and cytosol which results in an electrochemical potential across the inner membrane of the mitochondria, driving the production of Adenosine Triphosphate (ATP) as described in TI Karu, "Primary and secondary mechanisms of action of visible to near-IR radiation on cells. " J Photochem. Photobiol. B: Biol. 1999; 49:1-17.

As described in M Kato, K Shinizawa, S Yoshikawa "Cytochrome oxidase is a possible photoreceptor in mitochondria." Photobiochem. Photobiophys. 1981; 2: 263-269, ATP levels were raised after irradiation of HeLa cells at 632.8nm. The changes in the redox potential of the cell cytoplasm stimulate transient fluctuations in intracellular pH, and these changes are a necessary component involved in the transmission of mitogenic signals in the cell. This process is described as photosignal transduction and amplification. The flow of calcium ions (Ca²⁺) between the mitochondria and cytoplasm is also influenced by the changes in the respiratory chain. Ca²⁺ ions are the intracellular messengers in many biochemical processes and play a pivotal role in cell proliferation. These fluctuations in redox potential of the cell and in mono- and di-valent ions affect cell metabolism by influencing cyclic adenosine monophosphate (cAMP) levels. cAMP has been demonstrated to control both biosynthesis of DNA and RNA. Changes in intracellular cAMP concentrations following irradiation may help relate growth stimuli effects to the regulatory mechanisms of the proliferation activity of cells and therefore help in the process of revealing the mechanism of bio-stimulation by red light insofar as there is a relationship between cAMP, Ca²⁺ levels and the rate of DNA and RNA synthesis.

### Cellular reactions to infrared light at 830 nm

Unlike visible light energy, which penetrates into the cell and is absorbed in the organelles as discussed above, near infra-red (IR) light is absorbed by specific photoacceptors in the membranes of the cell and its organelles, including the nuclear membrane. The act of absorption induces vibrational and rotational changes in the molecules making up the irradiated membranes, which in turn leads to alterations of the membrane permeability. The primary reaction between IR energy and target cells is photophysical rather than photochemical. In the case of the cellular membrane, the altered permeability induces intra- and extra-cellular exchanges of components from the cytoplasm and extracellular fluid. The membrane Na-K pump is activated, inducing the synthesis of ATP to fuel this action. The mitochondria are further stimulated by the upward changes in the levels of Ca²⁺ ions and the presence in the cytosol of protons (H⁺) released from oxidative changes from, for example, ATP to ATPase and nicotinamide adenosine dinucleotide (NAD) to NAD⁺. Rising levels of NAD further fuel the upregulation of the cellular metabolism. Exactly the same photochemical cascade as seen in red light "biomodulation" of cells is thus induced. Based on Karu's model of red light-cell interaction already discussed, Smith KC. "The photobiological basis of low level laser radiation therapy. " Laser Therapy 1991; 3:19-24 postulated that indirect IR energy induction of the photochemical cascade from the photophysical response occurs in the target membrane and that the net effect of IR irradiation is the same as that of visible red energy, namely upregulation of the cellular metabolism which can result in a number of changes, including mitosis and enhancement of cellular function, as shown in Figure 1.

### Cellular mechanisms responsible for skin rejuvenation

Many of the cellular mechanisms involved in tissue rejuvenation are similar to those as a result of an inflammatory response. The mechanisms involve inflammation, proliferation, and remodelling (IPR) phases. It is the proliferative and remodelling phases which are responsible for generating new tissue such as collagen, elastin and other supportive components. Invoking an inflammatory response will kick-start the local tissue into this IPR chain, and ablative and non-ablative laser treatments inflict local trauma or wounds in the form of thermal damage to initiate this IPR chain. However, not only is this level of trauma greatly in excess of that required to initiate a cellular IPR response, it also results in a rapid proliferation of scar tissue in the body's attempt to seal the wound against infection and re-stabilise the connective tissue within the dermis which supports the overlying epidermis. This rapid proliferation of scar tissue can produce an unsatisfactory, relatively weak and inflexible fibrous matrix resulting in a tight but hard and inflexible skin structure.

It is not necessary to deliver such highly damaging levels of energy to the skin in order to invoke the IPR process via a macro wound response. Simply matching wavelengths with specific cellular targets responsible for initiating an inflammatory response will lead to a beneficial, aesthetic end-result without any of the trauma associated with thermal damage. In other words, it is better to let the tissue's cellular processes drive the regeneration process rather than an aggressive external insult.

Inflammation is an extremely vital initial component in the three-phase regeneration (IPR) chain. These are also the three phases of a wound healing process. Inflammation is key in non-ablative skin rejuvenation, including minimally invasive red LED phototherapy.

The phases of wound healing, and the cells involved must be fully understood in order to appreciate the important role of inflammation in these processes. These phases are shown in Figure 1. In the inflammation phase, which lasts for approximately 3-4 days, leukocytes peak, monocytes start to transform into phagocytes and mast cells peak and degranulate. This response initiates the migration of more macrophage cells and fibroblasts to the target stimulated by chemotactic signals from pre-existing fibroblasts, leukocytes and macrophages. The role of leukocytes and macrophages is primarily one of phagocytosis; however these cells also release fibroblast growth factor (FGF) stimulating those fibroblasts already present and those in migration. There is no clear transition between the inflammatory and proliferative phases, but more a gradual overlapping transition. Leukocytes decrease in number at the start of the second stage, macrophages continue to exist but gradually decrease, and by day 8-10 the number of fibroblasts peaks and starts to drop off. By day 18-20 (the end of the proliferative phase and the beginning of remodelling phase) the number of active fibroblasts falls off, and two transitional events occur: the further differentiation of active fibroblasts into active myofibroblasts and the de-differentiation of active fibroblasts into dormant fibrocytes. The role of the myofibroblasts is to position themselves on collagen fibres and exert a longitudinal force on the fibres, tightening and aligning them. This 'retuning' of the fibres and associated ExtraCellular Matrix (ECM) can take from 3 to 6 months.

Research, both in vitro and in vivo, has shown that red light and near infra red light has a biostimulatory effect on cellular metabolism. Cells function more efficiently and effectively in response to light between 600nm and 900nm, more so between 600nm to 700nm (red region) and 750-900nm (near infra-red region), but especially around 633nm and 670nm (red), and around 780nm, 830nm and 880nm (near infra-red). Red light at 633 nm has been shown to make mast cells preferentially degranulate, even when they are not attracted into the target area. Mast cells are always present in the dermis, located near blood vessels, and the stimulation given by their fast-acting proallergenic granules is seen by the surrounding tissue as 'inflammation', and so the wound healing process is started even without any thermal damage. Near infrared (IR) light also accelerates and strengthens the fibroblast-myofibroblast transformation. Light at 830 nm causes degranulation of mast cells even faster than 633 nm light. Near IR at 830 nm has also been shown to increase the chemotactic efficiency of both leukocytes and macrophage cells in recognising, moving to and engulfing their target, and also speeds up the internalisation process of whatever was phagocytosed, thereby releasing the cells back into the arena more quickly to perform their necessary function.

The inflammatory response from 633nm is a controlled short-lived phase which quickly transcends through to the proliferative phase, together with the creation of neovascularization through the photobiomodulation of haematopoietic stem cells in conjunction with the existing photoaltered endothelial cells increasing both the local blood and lymphatic vessel flow. In the case of the lymphatic drainage, this is extremely important in both transporting leukocytes and lymphocytes into the target area and maintaining the homeostasis of the treated skin. An increased blood supply not only raises the oxygen tension in the target area, creating gradients via which other cells can move efficiently enter the area, but also ensures that the connection between the papillary dermis and the basement membrane of the Dermal-Epidermal Junction (DEJ), and the basement membrane is supported.

Fibroblasts are essential to achieving the desired effect in the dermis during the second and third phases following the inflammatory reaction caused by the photomediated mast cell degranulation. The fibroblast is multifunctional, not only synthesising collagen and elastin, but also regulating the homeostasis of the ground substance and maintaining collagen fibres. 633 nm red light is extremely favourable in bringing about the appropriate photobiomodulatory response in irradiated fibroblasts.

However, 633nm has been shown to bring about a favourable reaction in all of the cell types involved in achieving the antiageing response. Hence the careful application of photobiological principles of light-tissue interaction improves clinical results.

LED phototherapy offers a new treatment modality, being non-invasive and safe with no patient downtime. However, the key to successful light therapy is the choice of the correct wavelength for photobiomodulation and the continuous delivery of the light in such a way as to maximise the light/photoacceptor interaction.

### The effect of 633nm and 830nm light on the wound healing process

Work by Kubota has demonstrated different wavelengths of light had differing degrees of effect in stimulating the cells involved in wound healing and hence the remodeling of the skin's supportive matrix, as shown in Figures 2 and 3. The wound healing process consists of three stages: Inflammation, Proliferation and Remodelling. During the inflammatory response, specific cells migrate to the target tissue area within specific timeframes. It was found possible, using suitable wavelengths of light, to effect and enhance the healing process.

For example, if a mild inflammatory response was evoked by the aggressive use of a facial scrub or mild microdermabrasion, the cells responsible for the inflammatory response were stimulated. During the inflammatory phase, leukocytes peaked, monocytes transformed into phagocytes and mast cells peaked and degranulated. This response initiated the migration of more macrophage cells and fibroblasts to the target stimulated by chemotactic signals from pre-existing fibroblasts, leukocytes and macrophages. At the start of proliferative phase macrophages gradually decreased, and the number of fibroblasts peaked and started to drop off. At the end of the proliferative phase two transitional events occurred: the differentiation of active fibroblasts into myofibroblasts and the de-differentiation of active fibroblasts into dormant fibrocytes. The role of the myofibroblasts, which are fibroblasts with a muscle fibre component, is to position themselves on collagen fibres and exert a longitudinal force on the fibres, tightening and aligning them.

It was possible to target specific cells using specific wavelengths as they appeared at the target site. When tissue was exposed to 830 nm, after a mild inflammatory initiation, this was found to stimulate optimally mast cells, leukocytes and macrophages. After 7 days, 633 nm was used to target optimally fibroblasts. At day 14 onwards, 830 nm was found to accelerate optimally the differentiation of fibroblasts to myofibroblasts.

Both red light at 633 nm and near infrared light at 830 nm accelerates the chemotactic and phagocytic activities of neutrophils and macrophage cells, and photomodulated activity of the macrophages has been shown to increase the synthesis of fibroblast growth factor (FGF) by a significant amount. Red light has been well recognized as enhancing local blood flow and lymphatic drainage, simultaneously increasing the oxygen tension in the treated area and the clearance of unwanted dermal debris. Red light not only targets the fibroblasts, but also the entire range of epidermal and dermal cells associated with maintaining the skin and stimulating blood flow. In addition, red light at 633 nm induces the degranulation of mast cells, which stimulates a primary athermal mild 'wounding' of tissue through the earlier release of the proinflammatory granules, followed by the beneficial effects on collagen synthesis and matrix maintenance by sodium dismutase granules, one of the body's most powerful antioxidants.

The effect of light at 830 nm in the near IR is also well documented. IR diode low level laser therapy (LLLT) has been used extensively for the management of wounds, stimulating the inflammatory response and speeding up the healing process. Tissue healing is a complex process that involves local and systemic responses and many cellular pathways, including those based on the following cell lines: mast cells, neutrophils, endotheliocytes, macrophages, fibroblasts and myofibroblasts. The importance of these cells in the remodelling of tissue after wound injury is well supported and forms the basis of the hypothesis that 830 nm will stimulate those cells involved in the wound healing process more effectively than 633 nm, with the exception of fibroblasts, and can thus influence the remodelling and restructuring of collagen. If the inflammatory response can be mimicked without invasion or damage to the skin then the use of 830 nm should greatly enhance the cellular response thereafter.

Enwemeka CS, Cohen-Kornberg E, Duswalt EP, Weber DM, and Rodriguez IM "Biomechanical effects of three different periods of GaAs laser photostimulation on tenotomized tendons ". Laser Therapy. 1994; 6: 181-188 has shown that near IR light accelerates and strengthens the fibroblast-myofibroblast transformation. el Sayed SO, Dyson M "Effect of laser pulse repetition rate and pulse duration on mast cell number and degranulation" Lasers Surg. Med. 1996; 19(4):433-7 shows that 830 nm causes degranulation of mast cells even faster than 633 nm light. Near IR at 830 nm has also been proved to increase the chemotactic efficiency of both leukocytes and macrophage cells in recognising, moving to and engulfing their target, and also speeds up the internalisation process of whatever was phagocytosed, thereby releasing the cells back into the arena more quickly to perform their necessary function.

The phases of wound healing, and the cells involved must be fully understood in order to appreciate the important role of inflammation in these processes. These phases are shown in Figure 2. In the inflammation phase, which lasts for approximately 3-4 days, leukocytes peak, monocytes start to transform into phagocytes and mast cells peak and degranulate. This response initiates the migration of more macrophage cells and fibroblasts to the target stimulated by chemotactic signals from pre-existing fibroblasts, leukocytes and macrophages. The role of leukocytes and macrophages is primarily one of phagocytosis; however these cells also release fibroblast growth factor (FGF) stimulating those fibroblasts already present and those in migration. There is no clear transition between the inflammatory and proliferative phases, but more a gradual overlapping transition. Leukocytes decrease in number at the start of the second stage, macrophages continue to exist but gradually decrease, and by day 8-10 the number of fibroblasts peaks and starts to drop off. By day 18-20 (the end of the proliferative phase and the beginning of remodelling phase) the number of active fibroblasts falls off, and two transitional events occur: the further differentiation of active fibroblasts into active myofibroblasts and the de-differentiation of active fibroblasts into dormant fibrocytes. The role of the myofibroblasts is to position themselves on collagen fibres and exert a longitudinal force on the fibres, tightening and aligning them. This 'retuning' of the fibres and associated ExtraCellular Matrix (ECM) can take from 3 to 6 months.

Research, both in vitro and in vivo, has shown that red light and near infra red light has a biostimulatory effect on cellular metabolism. Cells function more efficiently and effectively in response to light between 600nm and 900nm, more so between 600nm to 700nm (red region) and 750-900nm (near infra-red region), but especially around 633nm and 670nm (red), and around 780nm, 830nm and 880nm (near infra-red). Red light at 633 nm has been shown to make mast cells preferentially degranulate, even when they are not attracted into the target area. Mast cells are always present in the dermis, located near blood vessels, and the stimulation given by their fast-acting proallergenic granules is seen by the surrounding tissue as 'inflammation', and so the wound healing process is started even without any thermal damage. Near infrared (IR) light also accelerates and strengthens the fibroblast-myofibroblast transformation. Light at 830 nm causes degranulation of mast cells even faster than 633 nm light. Near IR at 830 nm has also been shown to increase the chemotactic efficiency of both leukocytes and macrophage cells in recognising, moving to and engulfing their target, and also speeds up the internalisation process of whatever was phagocytosed, thereby releasing the cells back into the arena more quickly to perform their necessary function.

The inflammatory response from 633nm is a controlled short-lived phase which quickly transcends through to the proliferative phase, together with the creation of neovascularization through the photobiomodulation of haematopoietic stem cells in conjunction with the existing photoaltered endothelial cells increasing both the local blood and lymphatic vessel flow. In the case of the lymphatic drainage, this is extremely important in both transporting leukocytes and lymphocytes into the target area and maintaining the homeostasis of the treated skin. An increased blood supply not only raises the oxygen tension in the target area, creating gradients via which other cells can move efficiently enter the area, but also ensures that the connection between the papillary dermis and the basement membrane of the Dermal-Epidermal Junction (DEJ), and the basement membrane is supported.

Fibroblasts are essential to achieving the desired effect in the dermis during the second and third phases following the inflammatory reaction caused by the photomediated mast cell degranulation. The fibroblast is multifunctional, not only synthesising collagen and elastin, but also regulating the homeostasis of the ground substance and maintaining collagen fibres. 633 nm red light is extremely favourable in bringing about the appropriate photobiomodulatory response in irradiated fibroblasts.

However, 633 nm has been shown to bring about a favourable reaction in all of the cell types involved in achieving the antiageing response. Hence the careful application of photobiological principles of light-tissue interaction improves clinical results.

### Embodiments of the Invention

Specific methods of treatment according to the invention will now be described. Further examples not being part of the invention are given to support the description of the embodiments.

### First Example

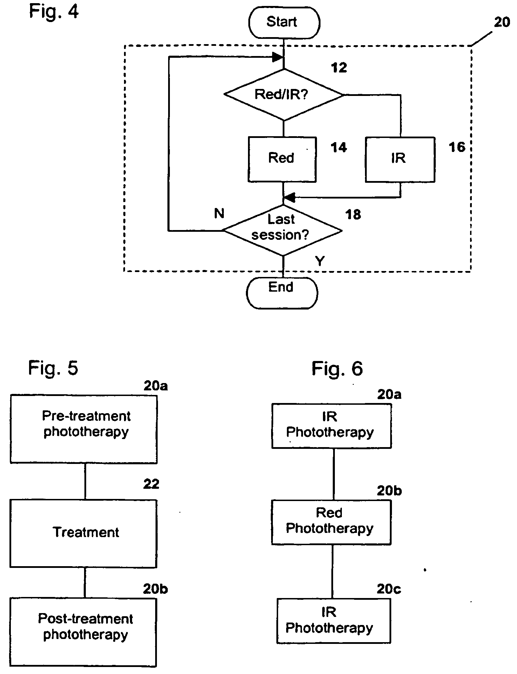

In a first example no being part of the invention, wound healing is accelerated by a course 20 of discrete phototherapy sessions each using a selected one of red or infrared light. A flow chart of the course is shown in Figure 4. For each session, a selection 12 is made as to whether that session will use red or infrared, according to a pre-determined programme. A phototherapy session using red light 14 or near infrared light 16 is then performed according to the selection 12. If the session is the last in the programme, as determined at step 18, then the course if finished; otherwise, the next session within the programme is performed, after an interval determined by the programme.

Visible red light, preferably with a wavelength around 633nm, may be applied directly to the wound bed, or over the area of interest in the case of a dermal wound under an intact epidermis due to its high effect on enhancing fibroblast function. Near-infrared light, preferably at 830 nm, may be used to increase the rate of the inflammatory response at the beginning of the wound healing process and in the final stages to accelerate the fibroblast to myofibroblast transformation. There is, therefore, a synergy obtained by using a combined red/infrared wavelength therapy to achieve "photobiomodulation" or "photoimmune modulation". For example, visible red light application may be delayed until two days after wounding (when the fibroblast are in their proliferation stage), but 830nm light may be applied to the wound immediately.

As illustrated in Figure 5, an aesthetic treatment 22 is enhanced by a first course of phototherapy 20a performed before the treatment 22 and a second course of phototherapy 20b performed after the treatment 22.

A combination of red (633nm) and infrared (830nm) light delivered pre- and post-laser ablation may reduce the downtime experienced by the patient. The use of temporally- and spectrally-selective combinational phototherapy (usually red and infrared light) can enhance aesthetic treatments which rely on photothermolysis, or mechanical damage via thermal ablation, coagulation, vaporisation, carbonisation or modification of tissue. This enhancement can result in an aesthetically improved result or appearance, reduced recovery time, and reduced exposure to infection or pain, thus benefiting the patient.

### Treatment Protocols

Examples of methods of treatment according to the first embodiment will now be described.

Each method includes phototherapy using red light and near-infrared light. Preferably, the red light is substantially monochromatic, with a wavelength in the range 600-700nm and most preferably around 633nm. Preferably, the infrared light is substantially monochromatic, with a wavelength in the range 800-910 nm and most preferably around 830 nm.

The intensity of light used is preferably no greater than 150 mW/cm², and preferably greater than 1 mW/cm². This range ensures efficacy of the treatment over a reasonable period of time, without causing further damage.

The total energy delivered per session is preferably no greater than 150 J/cm² and at least 1 J/cm².

Phototherapy may be used on the same day as an invasive treatment, but is preferably used on days preceding and following the day of the invasive treatment. The method of treatment preferably comprises a plurality of discrete, separate sessions, with each session comprising phototherapy with predominantly only one of red light or near-infrared light.

Examples of invasive treatment for which the above example may be applied include laser resurfacing, non-invasive photorejuvenation, intense light therapy (IPL), cosmetic breast surgery, plastic surgery, facial cosmetic surgery, reconstructive surgery, liposuction, surgical 'lifts', surgical implants, oto-, rhino- or blepharo-plasty, chemical peels, dermabrasion, sclerotherapy, hair transplant or scar reduction.

More specific examples will now be described in sequence. In these examples, each phototherapy session comprised phototherapy for a period of 20 minutes. Infrared (IR) phototherapy sessions used a wavelength of 830 nm with a total energy dose of 60 J/cm². Red phototherapy sessions used a wavelength of 633 nm with a total energy dose of 126 J/cm². However, it is not essential that each red or infrared session should use the same wavelength, period or energy dose.

Each of the examples includes an aesthetic treatment such as cosmetic surgery, laser ablation/resurfacing, laser therapy or non-invasive photorejuvenation.

### Example 1

i) Two weeks before treatment - three IR phototherapy sessions evenly spaced through one week
ii) One week before treatment - three red phototherapy sessions evenly spaced through one week
iii) Aesthetic treatment
iv) One IR phototherapy session per week for a period of 3 weeks after treatment.

### Example 2

i) One day before treatment - one IR phototherapy session
ii) Aesthetic treatment
iii) One day after treatment - one IR phototherapy session
iv) Two days after treatment - one IR phototherapy session
v) Thereafter - 4 red phototherapy sessions spaced 3 days apart.

### Example 3

i) Two days before treatment - one red phototherapy session
ii) One day before treatment - one red phototherapy session
iii) Aesthetic treatment
iv) Thereafter - one red phototherapy session per week for 2 weeks

### Example 4

i) Three days before treatment - one IR phototherapy session
ii) One day before treatment - one IR phototherapy session
iii) Aesthetic treatment
iv) One day after treatment - 1 IR phototherapy session
v) Two days after treatment - 1 IR phototherapy session
vi) 3 days after treatment - 1 red phototherapy session
vii) 6 days after treatment - 1 red phototherapy session
viii) 9 days after treatment - 1 IR phototherapy session
ix) 12 days after treatment - 1 IR phototherapy session

### Skin Rejuvenation

In the invention, methods of photorejuvenation of the skin are designed to stimulate different types of cell at different times so as to stimulate the IPR phases shown in Figure 2. Figure 3 shows that red visible light is optimum for stimulating fibroblasts, while near infrared is optimum for stimulating mast cells, leucocytes, macrophages and fibrocytes/myofibroblasts. Therefore, the preferred methods, as shown in Figure 6, comprise an initial course of treatment 20a with near-infrared light during the inflammation phase, followed by an intermediate course 20b of treatment using red light in the middle of the proliferation phase, and a final course 20c of treatment using near-infrared light during the remodelling phase.

The initial, intermediate and final courses may overlap in time to some degree, but it is preferable not to subject the skin to more energy than is necessary to achieve the desired cosmetic results. It is also preferable not to require the patient to attend more treatment sessions than are necessary. Therefore, the courses are preferably conducted sequentially, with little or no overlap.

In one treatment protocol, the skin of a patient requiring treatment for aged skin is subjected to a first course of treatment with near-infrared non-laser light having a narrow bandwidth. The wavelength of the light may be in the range 750-900 nm, preferably in the range 780 - 880 nm, and most preferably 800-850 nm. The course of treatment may last for 3 days to 2 weeks with 1 to 7 phototherapy sessions per week and not more than one session per day. In each treatment, the skin may initially be cleansed. The skin is then irradiated for 2 to 60 minutes with the light at an intensity of 1 to 150 mW/cm².

The skin is subsequently subjected to a second course of treatment with non-laser visible light having a narrow bandwidth. The wavelength is preferably in the range 600 to 700 nm, and most preferably 633nm. The course of treatment may last for 1 to 5 weeks with 1 to 7 phototherapy sessions per week and not more than one session per day. In each session, the skin may initially be cleansed. The skin is then irradiated for 2 to 60 minutes with the light at an intensity of 1 to 150 mW/cm².

The skin is then subjected to a third course of treatment with near-infrared non-laser light having a narrow bandwidth. The wavelength of the light may be in the range 750-900 nm, preferably in the range 780 - 880 nm, and most preferably 800-850 nm. The course of treatment may last for 1 to 10 weeks with 1 to 7 phototherapy sessions per week and not more than one session per day. In each treatment, the skin may initially be cleansed. The skin is then irradiated for 2 to 60 minutes with the light at an intensity of 1 to 150 mW/cm².

The third course may be omitted, but is advantageous in that it stimulates the remodelling phase.

Alternatively, the first and second courses can be alternated, since the first course uses the same wavelength as the third course, and may perform the dual role of stimulating remodelling and further inflammation.

### Combination light therapy (830/633nm) in facial skin rejuvenation

A more specific example of a treatment protocol in a clinical trial regime for facial skin rejuvenation will now be described.

### Week 1: Day 1, 3, 5, and repeated on Week 3, 4 and 5:

The facial skin is prepared by initial cleansing followed by exfoliation using a polyethylene-based exfoliant.

The subject's face is irradiated with LED light at 830nm for 20 minutes at ∼55 mW/cm² and 66 J/cm².

### Week 2: Day 8, 10 and 12

The facial skin is prepared by initial cleansing followed by exfoliation using a polyethylene-based exfoliant.

The subject's face is irradiated with LED light at 633nm for 20 minutes at ∼85mW/cm² and 96J/cm².

This treatment protocol has been found to give good results for facial skin rejuvenation. Alternative treatment protocols may nevertheless fall within the scope of the present invention.

### LED Light Source

The light source used for phototherapy is important, as it must first of all deliver photons of the correct wavelength to achieve targeting the appropriate cells, without delivering too much energy to the skin outside the correct wavelength, and deliver enough photons to these cells to realize the required photomodulation of the cellular function, in order to be clinically useful. Light sources comprising arrays of light-emitted diodes (LED's) are suitable for this purpose, as LED's are able to provide high intensity narrow bandwidth illumination with high efficiency and at low cost.

A suitable light source for the phototherapy sessions is illustrated in Figures 6 to 9. The light source comprises a base 2, an articulated arm 4 and a light-emitting head 6. The base 2 contains a power supply 3 for supplying electrical power to the light-emitting head 6, and a controller 5 for controlling the supply of power to the head 6. The controller 5 includes a switch and a timer for controlling the switch to determine the interval for which the head is switched on and emits light. The head may be switched on continuously over the interval, or may be pulsed on and off with a periodicity and duty cycle controlled by the controller 5. The interval, periodicity and duty cycle may be programmed into the controller 5 by a user by means of a keypad and display screen (not shown).

The articulated arm 4 is connected to the base 2 by a hinged joint 7a and is articulated along its length by further hinged joints 7b and 7c to give a sufficient degree of freedom in the position and angle of the head 6. The arm 4 carries a power connector from the controller 5 to the head 6.

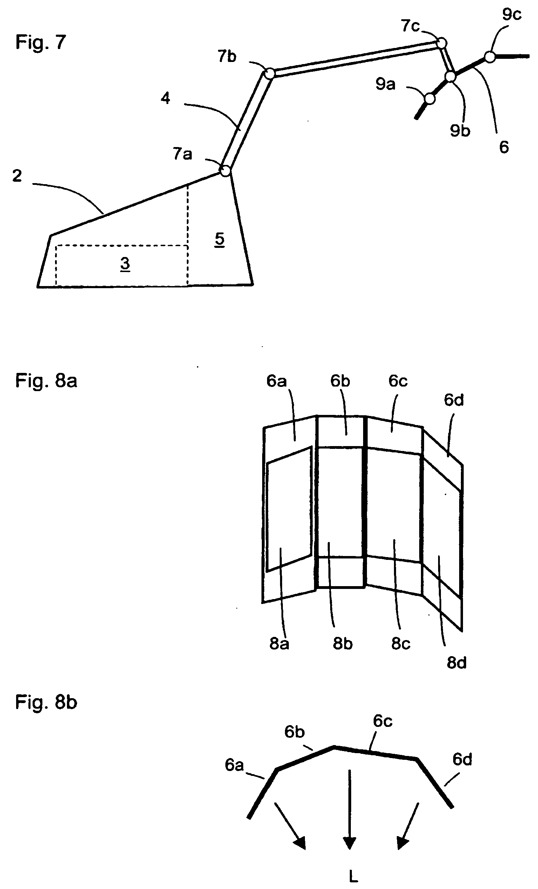

The head 6, as shown more particularly by Figure 7a, consists of a plurality rectangular panels 6a, 6b, 6c, 6d arranged side by side and joined at their edges by hinges 9a, 9b, 9c. Each panel 6 carries on its front face a corresponding matrix 8a, 8b, 8c, 8d of discrete light-emitting diodes (LED's). As shown in Figure 7b, the panels 6a-6d can be angled to form a concave surface such that light L emitted by the LED's is directed evenly on the skin to be treated.

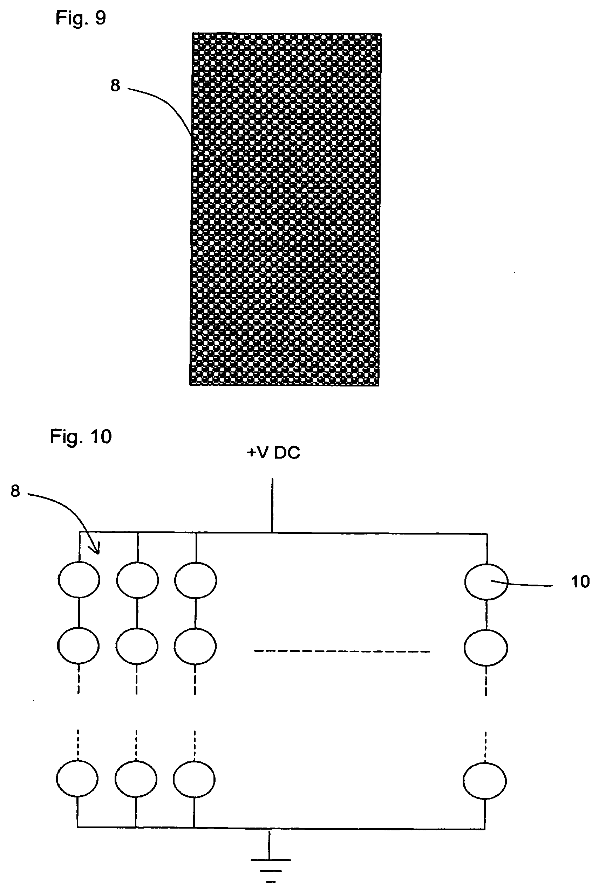

Figure 8 shows the physical arrangement of LED's in the matrix 8, while Figure 9 shows the series-parallel electrical connection between the LED's 10. A direct current (DC) voltage +V is applied across the matrix when power is supplied to head 6.

Interchangeable heads 6 may be provided, with each head carrying LED's which emit at a different wavelength. Thus, for use in the phototherapy sessions in any of the embodiments described above, a first head 6 may carry near-infrared-emitting LED's, and a second head 6 may carry red-emitting LED's. The controller may identify the head 6 currently fitted to the device, and indicate whether the currently fitted head is the correct one for the current phototherapy session. Thus, the controller 5 may be programmed with the durations, intervals and heads 6 required for the phototherapy sessions, and may thereby be arranged to implement the courses of treatment described with reference to any one of Figures 4 to 6. There may be provided a computer program for execution by the controller 5 so as to implement the course(s) of treatment.

Alternative light sources may be used, provided they are able to deliver narrow-bandwidth, non-laser light of the required wavelength.

## Claims

1. A non-therapeutic method of cosmetic treatment of aged skin of a patient, **characterised by** subjecting the skin to a first course of phototherapeutic treatment using infrared light having a wavelength in the range 800 to 910 nm and an intensity in the range 1 to 150 mW/cm² in daily treatment sessions within a period of between 3 days and 2 weeks; and followed by subjecting the skin to a second course of phototherapeutic treatment using red light having a wavelength in the range 600 to 700 nm and an intensity in the range 1 to 150 mW/cm² in daily treament sessions within a period of at least one week.

2. The method of claim 1, wherein the second course of phototherapeutic treatment last for a period of between 1 and 5 weeks.

3. The method of claim 1 or claim 2, further comprising subjecting the skin to a third course of phototherapeutic treatment using infrared light having a wave length in the range 750-900 nm and an intensity in the range 1 to 150 mW/cm² in daily treatment sessions within a period of at least 1 week.

4. The method of claim 3, wherein the third course lasts for a period of no more than 10 weeks.

5. The method of claim 1 or claim 2, further comprising repeating said first course and second course.

6. The method of claim 1 or claim 2, further comprising repeating alternately said first and second courses.

7. The method of any preceding claim, wherein the red light is monochromatic.

8. The method of any preceding claim, wherein the red light is non-laser light.

9. The method of any preceding claim, wherein the infrared light has a predominant wavelength of 830 nm.

10. The method of claim 9, wherein the red light has a predominant wavelength of 633 nm.

11. The method of any preceding claim, wherein the infrared light is monochromatic.

12. The method of any preceding claim, wherein the infrared light is non-laser light.

13. The method of claim 1, wherein the infrared light has a predominant wavelength within the range 800 to 850 nm.

14. The method of claim 1, wherein the infrared light has a predominant wavelength of 830 nm.

15. The method of any preceding claim, wherein each said treatment session lasts from 2 to 60 minutes.

16. The method of any preceding claim, wherein the infrared light is generated by a plurality of light emitting diodes.

17. The method of claim 16, wherein the light-emitting diodes are arranged in one or more arrays arranged to provide substantially uniform illumination of the skin.

18. The method of any preceding claim, wherein the red light is generated by a plurality of light emitting diodes.

19. The method of claim 18, wherein the light-emitting diodes are arranged in one or more arrays arranged to provide substantially uniform illumination of the skin.

## Patentansprüche

1. Nicht-therapeutisches Verfahren zur kosmetischen Behandlung gealterter Haut eines Patienten, **dadurch gekennzeichnet, dass** die Haut einer Infrarotlicht mit einer Wellenlänge im Bereich 800 bis 910 nm und einer Intensität im Bereich 1 bis 150 mW/cm² nutzenden ersten lichttherapeutischen Behandlungskur in täglichen Behandlungssitzungen in einem Zeitraum von zwischen 3 Tagen und 2 Wochen ausgesetzt wird; und darauf folgend die Haut einer Rotlicht mit einer Wellenlänge im Bereich 600 bis 700 nm und einer Intensität im Bereich 1 bis 150 mW/cm² nutzenden zweiten lichttherapeutischen Behandlungskur in täglichen Behandlungssitzungen in einem Zeitraum von mindestens einer Woche ausgesetzt wird.

2. Verfahren nach Anspruch 1, wobei die zweite lichttherapeutische Behandlungskur für einen Zeitraum von zwischen 1 und 5 Wochen andauert.

3. Verfahren nach Anspruch 1 oder Anspruch 2, weiter umfassend, dass die Haut einer Infrarotlicht mit einer Wellenlänge im Bereich 750-900 nm und einer Intensität im Bereich 1 bis 150 mW/cm² nutzenden dritten lichttherapeutischen Behandlungskur in täglichen Behandlungssitzungen in einem Zeitraum von mindestens 1 Woche ausgesetzt wird.

4. Verfahren nach Anspruch 3, wobei die dritte Kur für einen Zeitraum von nicht mehr als 10 Wochen andauert.

5. Verfahren nach Anspruch 1 oder Anspruch 2, weiter umfassend das Wiederholen der ersten Kur und der zweiten Kur.

6. Verfahren nach Anspruch 1 oder Anspruch 2, weiter umfassend das abwechselnde Wiederholen der ersten und der zweiten Kur.

7. Verfahren nach einem der vorangehenden Ansprüche, wobei das Rotlicht monochromatisch ist.

8. Verfahren nach einem der vorangehenden Ansprüche, wobei es sich bei dem Rotlicht um Nicht-Laserlicht handelt.

9. Verfahren nach einem der vorangehenden Ansprüche, wobei das Infrarotlicht eine vorherrschende Wellenlänge von 830 nm aufweist.

10. Verfahren nach Anspruch 9, wobei das Rotlicht eine vorherrschende Wellenlänge von 633 nm aufweist.

11. Verfahren nach einem der vorangehenden Ansprüche, wobei das Infrarotlicht monochromatisch ist.

12. Verfahren nach einem der vorangehenden Ansprüche, wobei es sich bei dem Infrarotlicht um Nicht-Laserlicht handelt.

13. Verfahren nach Anspruch 1, wobei das Infrarotlicht eine vorherrschende Wellenlänge im Bereich 800 bis 850 nm aufweist.

14. Verfahren nach Anspruch 1, wobei das Infrarotlicht eine vorherrschende Wellenlänge von 830 nm aufweist.

15. Verfahren nach einem der vorangehenden Ansprüche, wobei jede Behandlungssitzung von 2 bis 60 Minuten andauert.

16. Verfahren nach einem der vorangehenden Ansprüche, wobei das Infrarotlicht von mehreren Leuchtdioden erzeugt wird.

17. Verfahren nach Anspruch 16, wobei die Leuchtdioden in einer oder mehr Gruppierungen angeordnet sind, um im Wesentlichen gleichmäßige Beleuchtung der Haut vorzusehen.

18. Verfahren nach einem der vorangehenden Ansprüche, wobei das Rotlicht von mehreren Leuchtdioden erzeugt wird.

19. Verfahren nach Anspruch 18, wobei die Leuchtdioden in einer oder mehr Gruppierungen angeordnet sind, um im Wesentlichen gleichmäßige Beleuchtung der Haut vorzusehen.

## Revendications

1. Procédé non thérapeutique de traitement cosmétique de la peau marquée par l'âge d'un patient, **caractérisé par** l'administration d'une première série de traitements photothérapeutiques sur la peau en utilisant une lumière infrarouge ayant une longueur d'onde dans la plage de 800 à 910 nm et une intensité dans la plage de 1 à 150 mW/cm² à raison de sessions de traitement quotidiennes durant une période entre 3 jours et 2 semaines ; puis l'administration d'une deuxième série de traitements photothérapeutiques sur la peau en utilisant une lumière rouge ayant une longueur d'onde dans la plage de 600 à 700 nm et une intensité dans la plage de 1 à 150 mW/cm² à raison de sessions de traitement quotidiennes durant une période d'au moins une semaine.

2. Procédé selon la revendication 1, dans lequel la deuxième série de traitements photothérapeutiques dure pendant une période entre 1 et 5 semaines.

3. Procédé selon la revendication 1 ou la revendication 2, comprenant en outre l'administration d'une troisième série de traitements photothérapeutiques sur la peau en utilisant une lumière infrarouge ayant une longueur d'onde dans la plage de 750 à 900 nm et une intensité dans la plage de 1 à 150 mW/cm² à raison de sessions de traitement quotidiennes durant une période d'au moins 1 semaine.

4. Procédé selon la revendication 3, dans lequel la troisième série dure pendant une période ne dépassant pas 10 semaines.

5. Procédé selon la revendication 1 ou la revendication 2, comprenant en outre la répétition desdites première série et deuxième série.

6. Procédé selon la revendication 1 ou la revendication 2, comprenant en outre la répétition alternée desdites première et seconde séries.

7. Procédé selon l'une quelconque des revendications précédentes, dans lequel la lumière rouge est monochrome.

8. Procédé selon l'une quelconque des revendications précédentes, dans lequel la lumière rouge est une lumière non-laser.

9. Procédé selon l'une quelconque des revendications précédentes, dans lequel la lumière infrarouge a une longueur d'onde prédominante de 830 nm.

10. Procédé selon la revendication 9, dans lequel la lumière rouge a une longueur d'onde prédominante de 633 nm.

11. Procédé selon l'une quelconque des revendications précédentes, dans lequel la lumière infrarouge est monochrome.

12. Procédé selon l'une quelconque des revendications précédentes, dans lequel la lumière infrarouge est une lumière non-laser.

13. Procédé selon la revendication 1, dans lequel la lumière infrarouge a une longueur d'onde prédominante dans la plage de 800 à 850 nm.

14. Procédé selon la revendication 1, dans lequel la lumière infrarouge a une longueur d'onde prédominante de 830 nm.

15. Procédé selon l'une quelconque des revendications précédentes, dans lequel chaque dite session de traitement dure de 2 à 60 minutes.

16. Procédé selon l'une quelconque des revendications précédentes, dans lequel la lumière infrarouge est générée par une pluralité de diodes électroluminescentes.

17. Procédé selon la revendication 16, dans lequel les diodes électroluminescentes sont disposées en une ou plusieurs matrices agencées pour éclairer sensiblement uniformément la peau.

18. Procédé selon l'une quelconque des revendications précédentes, dans lequel la lumière rouge est générée par une pluralité de diodes électroluminescentes.

19. Procédé selon la revendication 18, dans lequel les diodes électroluminescentes sont disposées en une ou plusieurs matrices agencées pour éclairer sensiblement uniformément la peau.
